# EUROPEAN PATENT SPECIFICATION

(11) **EP 3 603 659 B1**
(45) Date of publication and mention of the grant of the patent: **08.10.2025**
(21) Application number: 18778300.6
(22) Date of filing: 27.03.2018
(51) Int. Cl.: A61K 38/16, A61K 9/00, A61P 11/00, A61P 11/02

(54) **PHARMACEUTICAL COMPOSITION FOR USE IN PREVENTION OR TREATMENT OF INFLAMMATORY RESPIRATORY DISEASE, WITH FUSION PROTEIN OF CELL PERMEABLE PEPTIDE AND CTCTLA4 PEPTIDE CONTAINED AS EFFECTIVE INGREDIENT THEREIN**
PHARMAZEUTISCHE ZUSAMMENSETZUNG ZUR PRÄVENTION ODER BEHANDLUNG VON ENTZÜNDLICHEN ATEMWEGSERKRANKUNGEN MIT FUSIONSPROTEIN AUS ZELLDURCHLÄSSIGEM PEPTID UND CTCTLA4-PEPTID ALS WIRKSTOFF DARIN
COMPOSITION PHARMACEUTIQUE DE PRÉVENTION OU TRAITEMENT DE MALADIE RESPIRATOIRE INFLAMMATOIRE, À PROTÉINE DE FUSION DE PEPTIDE PERMÉABLE AUX CELLULES ET DE PEPTIDE CTCTLA4, CONTENUE EN TANT QUE PRINCIPE ACTIF DANS CETTE COMPOSITION

(30) Priority: 27.03.2017 KR 20170038612
(43) Date of publication of application: 05.02.2020
(73) Proprietor: IUCF-HYU (Industry-University Cooperation Foundation Hanyang University), Seoul 04763 (KR)
(72) Inventor: CHOI, Je-Min, Seoul 03716 (KR); LIM, Sangho, Seoul 04939 (KR)
(74) Representative: dompatent von Kreisler Selting Werner - Partnerschaft von Patent- und Rechtsanwälten mbB
(86) International application number: PCT/KR2018/003622
(87) International publication number: WO 2018/182301

(56) References cited:
- KR-A- 20080 084 937
- KR-A- 20170 022 943
- KR-A- 20170 022 943
- US-A1- 2007 105 775
- US-A1- 2007 105 775
- US-A1- 2014 322 183
- CHOI JE-MIN ET AL: "Intranasal delivery of the cytoplasmic domain of CTLA-4 using a novel protein transduction domain prevents allergic inflammation", NATURE MEDICINE, NATURE PUB. CO, NEW YORK, vol. 12, no. 5, 1 May 2006 (2006-05-01), pages 574 - 579, XP002530023, ISSN: 1078-8956, [retrieved on 20060409], DOI: 10.1038/NM1385
- SANGHO LIM ET AL: "dNP2 is a blood-brain barrier-permeable peptide enabling ctCTLA-4 protein delivery to ameliorate experimental autoimmune encephalomyelitis", NATURE COMMUNICATIONS, vol. 6, 15 September 2015 (2015-09-15), pages 8244, XP055368583, DOI: 10.1038/ncomms9244
- LUZINA IRINA ET AL: "Regulation of inflammation by interleukin-4: a review of "alternatives"", vol. 92, no. 4, 1 October 2012 (2012-10-01), pages 753 - 764, XP009522898, ISSN: 0741-5400, Retrieved from the Internet <URL:https://www.ncbi.nlm.nih.gov/pmc/articles/PMC3441310/> DOI: 10.1189/JLB.0412214
- LIM, S. ET AL.: "dNP2 Is a Blood-brain Barrier-permeable Peptide Enabling ctCTLA-4 Protein Delivery to Ameliorate Experimental Autoimmune Encephalomyelitis", NATURE COMMUNICATIONS, vol. 6, 15 September 2015 (2015-09-15), pages 1 - 13, XP055368583
- NIE, W. ET AL.: "Cytotoxic T-lymphocyte Associated Antigen 4 Polymorphisms and Asthma Risk: a Meta-analysis", PLOS ONE, vol. 7, no. 7, 26 July 2012 (2012-07-26), pages e42062, XP055559865
- LIM, S. ET AL.: "dNP2-ctCTLA-4 Inhibits German Cockroach Extract-induced Allergic Airway Inflammation and Hyper-responsiveness via Inhibition of Th2 Responses", EXPERIMENTAL & MOLECULAR MEDICINE, vol. 49, no. 8, 4 August 2017 (2017-08-04), pages e362, XP055559867

## Description

### [Background Art]

The present disclosure relates to a pharmaceutical composition for preventing or treating an inflammatory respiratory disease, which contains a fusion protein in which a cell-penetrating peptide and a ctCTLA4 peptide are fused as an active ingredient, more particularly to a pharmaceutical composition which is capable of exhibiting superior effect of preventing or treating an inflammatory respiratory disease through nasal mucosa without having to be administered directly into the lung or bronchus as cell permeability is improved and targeting effect for the inflammatory respiratory disease is enhanced through fusion of the dNP2 peptide and the ctCTLA4 peptide.

### [Background Art]

Inflammatory respiratory diseases are fatal chronic lung diseases caused by dust mite, pollen, cockroach, etc. and cause severe health problems such as bad breath, difficulty in breathing, cough, wheezing, chest tightness and pain.

German cockroach is one of the major causes of allergic diseases. It is known to cause inflammatory allergic airway disease by inducing sensitive T cell responses. German cockroach extract (GCE) has been widely used to construct allergic inflammation models.

For treatment of allergic reactions, a variety of immunoregulatory biological molecules such as antibodies mediating allergic reactions have been proposed as therapeutic agents. Among them, T cell-specific immunoregulators are problematic in that topical administration, e.g., intranasal administration, for treatment of asthma is difficult and only systemic administration is possible.

Meanwhile, monoclonal antibodies developed for treatment of asthma such as anti-IgE (omalizumab), anti-IL-5 (mepolizumab), anti-IL-13 (anrukinzumab) and anti-TGF-β (daclizumab) also have the limitation that only systemic administration is possible and have the problems associated with the systemic administration, such as side effects, vulnerability to infection and high cost.

In addition, the antibodies are problematic in that only systemic administration is possible due to their tight binding in the respiratory system and limitation in biological sizes.

The inventors of the present disclosure have studied on intranasal administration of a fusion protein instead of administration directly into the bronchus or systemic administration and have completed the present disclosure.

Patent document 1: Korean Patent Publication No. 10-2008-0066962.
CHOI JE-MIN ET AL: "Intranasal delivery of the cytoplasmic domain of CTLA-4 using a novel protein transduction domain prevents allergic inflammation", NATURE MEDICINE, NATURE PUB. CO, NEW YORK, vol. 12, no. 5, 1 May 2006 (2006-05-01), pages 574-579 describes the prevention of allergic inflammation/allergic asthma through intranasal delivery of the fusion protein Hph1-CTLA-4p US 2007/7105775 describes a method for inhibiting T cell activation in an autoimmune or inflammatory disease such as asthma, comprising administering a fusion polypeptide comprising a protein transduction domain such as Hph1 and a cytoplasmic domain of a receptor protein such as ctCTLA-4.
SANGHO LIM ET AL: "dNP2 is a blood-brain barrier-permeable peptide enabling ctCTLA-4 protein delivery to ameliorate experimental autoimmune encephalomyelitis", NATURE COMMUNICATIONS, vol. 6, 15 September 2015 (2015-09-15), page 8244 and KR 2017 0022943 A describe that the injection of the claimed fusion protein dNP2-ctCTLA-4 ameliorated experimental autoimmune encephalomyelitis, i.e. multiple sclerosis.
LUZINA IRINA ET AL: "Regulation of inflammation by interleukin-4: a review of "alternatives"", JOURNAL OF LEUKOCYTE BIOLOGY, JOHN WILEY & SONS LTD, GB vol. 92, no. 4, 1 October 2012 (2012-10-01), pages 753-764, describes the implications of IL-4 and IL-13 in inflammation.

### [Disclosure]

### [Technical Problem]

The present disclosure is directed to providing a pharmaceutical composition which exhibits superior delivery and targeting efficiency for a bronchus and lung cells by administering a fusion protein via an intranasal route, unlike the existing administration routes, and has remarkably improved activities of suppressing cytokine expression, suppressing airway inflammation, alleviating airway hyperresponsiveness to allergens, alleviating Th2 inflammation, etc.

### [Technical Solution]

The invention is set out in the appended set of claims.

### [Advantageous Effects]

The present disclosure can provide quick, fast, and effective therapeutic or preventive effect for inflammatory respiratory diseases, particularly allergic asthma, via administration through the respiratory system, with which effective therapeutic or preventive effect could not be achieved, by providing superior delivery and targeting efficiency for a bronchus and lung cells and remarkably improved activities of suppressing cytokine expression, suppressing airway inflammation, alleviating airway hyperresponsiveness to allergens, alleviating Th2 inflammation, etc. as compared to the existing therapeutic agents.

In addition, the present disclosure is potentially applicable to new therapies and prescriptions because the therapeutic agent can be administered topically at high concentrations with decreased side effects, unlike the existing therapeutic agents.

### [Brief Description of Drawings]

FIG. 1 shows the experimental conditions (administration time, administration dosage and administration route for each group) of Test Example 1.
FIG. 2 shows a result of preparing frozen lung tissue sections (8 µm) from each control group and test group of Test Example 1, staining cell nuclei with Hoechst and observing by fluorescence microscopy.
FIG. 3 shows a flow cytometry result of fluorescence signals from the bronchoalveolar lavage fluid of each control group and test group of Test Example 1. The red fluorescence histograms and mean fluorescence intensities (MFI) are shown.
FIG. 4 shows the experimental conditions (administration time, administration dosage and administration route for each group) of Test Example 2.
FIG. 5 and FIG. 6 show a result of analyzing airway hyperresponsiveness (AHR) to methacholine for each test group and control group of Test Example 2. FIG. 5 shows a result of measuring airway resistance depending on methacholine concentration for each test group and control group of Test Example 2. FIG. 6 shows a result of measuring airway elastance depending on methacholine concentration for each test group and control group of Test Example 2. The representative results for each group (n = 5) are presented as mean ± s.e.m. (standard error of mean). **p < 0.01, ***p < 0.001.
FIG. 7 shows a result of observing lung tissue for each test group and control group of Test Example 3. The lung tissue of each test group and control group of Test Example 3 was imaged after staining with PAS. The red region indicates mucus-secreting goblet cells.
FIG. 8 shows a result of analyzing the number of mucus-secreting goblet cells in FIG. 7 using the ImageJ 1.50i software.
FIG. 9 shows a result of observing lung tissue for each test group and control group of Test Example 3. The lung tissue of each test group and control group of Test Example 3 was imaged after staining with Masson's trichrome stain. The blue region indicates collagen deposition and fibrosis.
FIG. 10 shows a result of analyzing the fibrotic area of FIG. 9 using the ImageJ 1.50i software. The representative results for each group (n = 5) are presented as mean ± s.e.m. (standard error of mean). ***p < 0.001.
FIG. 11 shows a result of cytocentrifuging a bronchoalveolar lavage fluid recovered from each test group and control group of Test Example 4 on a slide and counting the number of total cells (a), macrophages (b) and eosinophils (c) after staining using the Hemacolor staining kit.

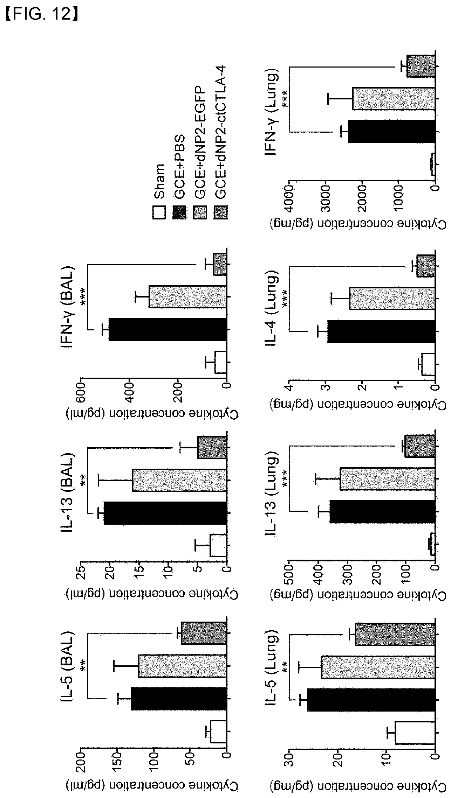

FIG. 12 shows a result of analyzing the expression level of IL-5, IL-13 and IFN-γ in a supernatant of a bronchoalveolar lavage (BAL) using the ELISA kit (top) and a result of analyzing the expression level of IL-5, IL-13, IL-4 and IFN-γ in the frozen lung tissue using the ELISA kit (bottom). The concentration of IL-5, IL-13, IL-4 and IFN-γ was analyzed with the ELSISA kit and the result was normalized to the total protein concentration of the tissue. The protein concentration was quantified by the Bradford assay. n = 5 per each group and the results are presented as mean ± s.e.m. (standard error of mean). *p < 0.05, **p < 0.01.
FIG. 13 shows the experimental conditions (administration time, administration dosage and administration route for each group) of Test Example 5.

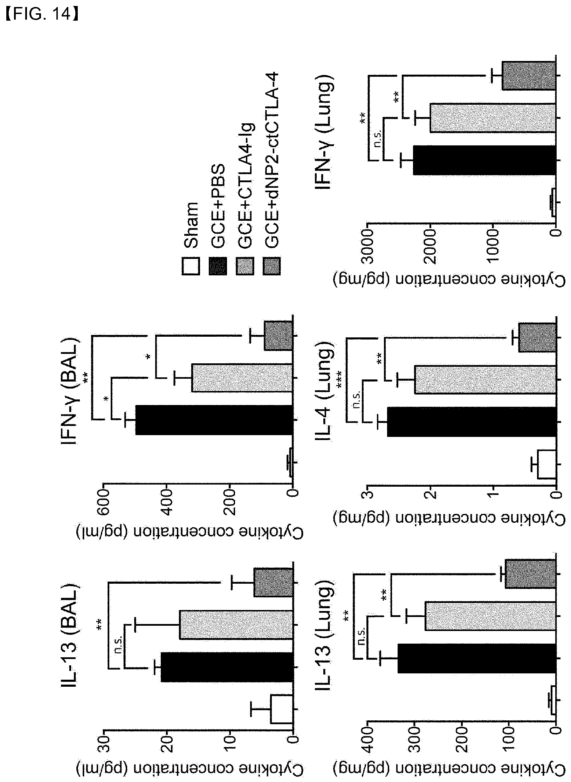

FIG. 14 shows a result of analyzing the expression level IL-13 and IFN-γ in a supernatant of a bronchoalveolar lavage (BAL) using the ELISA kit (top) and a result of analyzing the expression level of IL-13, IL-4 and IFN-γ in the frozen lung tissue using the ELISA kit (bottom). The concentration of IL-13, IL-4 and IFN-γ was analyzed with the ELSISA kit and the result was normalized to the total protein concentration of the tissue. The protein concentration was quantified by the Bradford assay. n = 5 per each group and the results are presented as mean ± s.e.m. (standard error of mean). *p < 0.05, **p < 0.01.
FIG. 15 shows microscopic images of a lung tissue recovered from each test group and control group of Test Example 5 imaged after staining with H&E (hematoxylin and eosin). The representative images for each group (n = 5) are shown.
FIG. 16 shows a result of measuring the airway epithelial thickness of FIG. 15 using the ImageJ 1.50i software. The result is presented as mean ± s.e.m. (standard error of mean). *p < 0.05, **p < 0.01.
FIG. 17 shows a result of analyzing the differentiation of Th2 cells by non-activated, +PBS, +dNP2-EGFP and +dNP2-ctCTLA-4.
FIG. 18 shows a result of calculating the percentage (%) of cytokines produced in CD4⁺IL-4⁺ differentiated Th2 cells. The result is presented as mean ± s.e.m. (standard error of mean) (n = 3). **p < 0.01. NA (non-activated) denotes a negative control group, not stimulated by cytokines or anti-TcR antibody.

### [Best Mode]

Hereinafter, various aspects and exemplary embodiments of the present disclosure are described in more detail.

An aspect of the present disclosure relates to a pharmaceutical composition for use through intranasal administration in preventing or treating an inflammatory respiratory disease, comprising as an active ingredient a protein in which a cell-penetrating peptide dNP2 of SEQ ID NO 1 (KIKKVKKKGRKGSKIKKVKKKGRK) and a ctCTLA4 peptide of SEQ ID NO 2 (KMLKKRSPLTTGVYVKMPPTEPECEKQFQPYFIPIN) are fused. In the present disclosure, the "fusion protein" refers to a covalently bonded complex which contains the cell-penetrating peptide of SEQ ID NO 1 and the ctCTLA4 peptide of SEQ ID NO 2 formed through genetic fusion or chemical bonding.

The "genetic fusion" refers to linkage through linear covalent bonding by way of genetic expression of a DNA sequence encoding a protein.

CTLA4, generally known as cytotoxic T-lymphocyte antigen-4, is an important immunoprotein suppressing T cell activation. CTLA-4 forms binding with a costimulatory molecule on the cell surface where antigens such as CD80 and CD86 are present, which is much more effective than the binding with the costimulatory receptor CD28 of T cells. This interaction results in negative signals and suppresses the T cells. Abatacept is a recombinant protein composed of the immunoglobulin G constant region CTLA-4-Ig fused to the extracellular domain of CTLA-4. It is effective in suppressing allergic airway inflammation in an OVA (ovalbumin)-induced asthma animal model through eosinophil infiltration, Th2 cytokine expression and decreased serum IgE via intravenous or intraabdominal injection.

Recently, new human-derived cell-penetrating peptides capable of accessing resident cells of various tissues including the brain tissue by effectively circumventing blood vessels have been developed. An example is the recombinant protein dNP2-ctCTLA4 composed of the dNP2 peptide and the cytoplasmic domain of CTLA-4.

However, only the preventive or therapeutic effect of autoimmune encephalitis was identified in mice with Th1 and Th17 cells decreased in the spinal cord. The present disclosure was completed by finding that a therapeutic or preventive effect for entirely different diseases as compared to the existing method can be achieved by administering the dNP2-ctCTLA-4 fusion protein through a totally different route (intranasal route). The administration of the dNP2-ctCTLA-4 fusion protein through the intranasal route provides a therapeutic or preventive effect for an inflammatory respiratory disease unrelated with the central nervous system, specifically allergic asthma. When the dNP2-ctCTLA-4 fusion protein is inhaled through the intranasal route in the form of a spray or a powder, the protein can be effectively delivered to lung resident cells and the delivered dNP2-ctCTLA-4 fusion protein exerts a preventive or therapeutic effect for the inflammatory respiratory disease by controlling Th2 inflammation effectively.

It was found out that, if the dNP2 peptide in the dNP2-ctCTLA-4 fusion protein is replaced with other cell-penetrating peptides, the protein cannot be delivered effectively into the airway, bronchus or lungs when administered through the intranasal route and it cannot effectively targeted to the lung resident cells.

That is to say, the administration of the dNP2-ctCTLA-4 fusion protein through the intranasal route provides a remarkably superior pharmacological effect (2-3 times or higher) for allergic asthma as compared to the existing CTLA-4-Ig and also provides a pharmacological effect distinguished from that of the existing dNP2-ctCTLA-4.

Through a test example of the present disclosure, it was confirmed that the administration of the dNP2-ctCTLA-4 fusion protein to a GCE (German cockroach extract)-induced allergic asthma animal model provides an effect of suppressing hyperresponsiveness and Th2 inflammation.

The inventors investigated the inhibitory effect of the dNP2-ctCTLA-4 fusion protein on the airway inflammation induced by cockroach. It was found out that the dNP2-ctCTLA-4 fusion protein is effectively delivered to the lung cells by being administered into the airway or bronchus through the intranasal route. In addition, it was confirmed that the dNP2-ctCTLA-4 fusion protein can be effectively delivered to the resident cells by passing through the respiratory epithelium. In other words, the dNP2-ctCTLA-4 fusion protein according to the present disclosure can effectively control allergic asthma by alleviating airway hyperresponsiveness, Th2 inflammation and airway remodeling and directly inhibiting Th2 differentiation when administered through the intranasal route. In addition, it was found out that the dNP2-ctCTLA-4 fusion protein according to the present disclosure achieved 2-3 times or higher pharmacological effect as compared to the existing CTLA-4-Ig.

When the dNP2-ctCTLA-4 fusion protein according to the present disclosure is administered through a route other than the intranasal route, the pharmacological effect for the inflammatory respiratory disease of the present disclosure cannot be attained.

The peptide having IL-2 inhibitory activity of an amino acid sequence represented by SEQ ID NO 2 used in the present disclosure is a sequence derived from the cytoplasmic domain of the CTLA-4 protein, obtained by encoding a part of the exon 4 of the full-length CTLA-4 protein obtained from human or mouse. It is an amino acid sequence corresponding to 100% correspondence in the amino acid sequences of the CTLA-4 proteins of human and mouse. It is less likely to cause side effects such as immune responses when applied to the human body.

The peptide having IL-2 inhibitory activity of an amino acid sequence represented by SEQ ID NO 2 is a fragment of the CTLA-4 protein which exhibits IL-2 expression inhibitory activity. It can pass through the central nervous system, i.e., the blood-brain barrier or the blood-spinal cord barrier, with high efficiency and can be used both for human and non-human animal (mouse) because it has a 100% identical amino acid sequence.

Specifically, the peptide having IL-2 inhibitory activity of an amino acid sequence represented by SEQ ID NO 2 contains an amino acid sequence from the 188th amino acid residue to the 213rd amino acid residue of the cytotoxic T-lymphocyte antigen-4 (CTLA-4) protein (SEQ ID NO 2) and is also referred to as 'ctCTLA-4' hereinafter. It is a polypeptide with the N-terminal and C-terminal partly deleted to provide permeation activity for the nasal mucosa, bronchial mucosa or lung epithelial cells when administered through an intranasal route and to provide preventive or therapeutic effect for an inflammatory respiratory disease.

The ctCTLA4 peptide may be extracted from a natural product, synthesized or prepared by a genetic recombination method based on a DNA sequence.

The inflammatory respiratory disease may be selected from a group consisting of asthma, chronic obstructive pulmonary disease (COPD), acute lung injury, pyothorax, lung abscess, pneumonia, pulmonary tuberculosis, bronchitis, sore throat, tonsillitis, paranasal sinusitis, rhinitis, constrictive bronchiolitis and laryngitis.

Examples of the asthma may include bronchial asthma, atopic asthma, atopic bronchial IgE-mediated asthma, non-atopic asthma, allergic asthma, non-allergic asthma, etc. and examples of the bronchitis may include acute bronchitis, chronic bronchitis, bronchiolitis, catarrhal bronchitis, etc.

The allergic asthma may be an allergic asthma caused by dust mite, pollen, animal fur or dander, cockroach, food, drug, flu, cigarette smoke, indoor contamination, air pollution, food additive, exercise, climate change, yellow dust or stress. More specifically, it may be an allergic asthma caused by cockroach.

Specifically, the pharmaceutical composition of the present disclosure may be injected only through an intranasal route. When it is administered through other administration routes such as intravenous injection, subcutaneous injection, intramuscular injection, intraabdominal injection or transdermal administration, the pharmacological effect for an inflammatory respiratory disease, particularly allergic asthma, cannot be achieved.

The composition of the present disclosure may further contain an adequate carrier, excipient and diluent commonly used to prepare a pharmaceutical composition. The formulation of the pharmaceutical composition according to the present disclosure is not specially limited as long as it can be administered through an intranasal route. In the present disclosure, the "administration through an intranasal route" includes any mode of administration allowing the pharmaceutical composition to be administered to the airway, trachea, bronchus or pulmonary alveoli through the nasal cavity by being inhaled in the form of a solution, a powder, an aerosol or a sprayable solution or powder regardless of the addition of a stabilizer or other excipients.

Specifically, as suitable formulations known in the art, those disclosed in Remington's Pharmaceutical Sciences (Mack Publishing Company, Easton PA) may be used.

Specifically, for the administration through an intranasal route, a pressurized sprayer using a compressed air/oxygen mixture, an ultrasonic sprayer, an electric micropump sprayer, a metered-dose inhaler (MDI) or a dry powder inhaler (DPI) may be used. An aerosol may be delivered by mechanical ventilation or via an endotracheal tube inserted in a patient.

Examples of the carrier, excipient and diluent that may be contained in the pharmaceutical composition of the present disclosure include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate and mineral oil. For the formulation, a commonly used diluent or excipient such as a filler, an extender, a binder, a wetting agent, a disintegrant, a surfactant, etc. is used.

The term "administration" used in the present disclosure refers to provision of the composition of the present disclosure to a subject by any appropriate method.

The specific administration dosage of the pharmaceutical composition of the present disclosure varies depending on the condition and body weight of a subject, the severity of a disease, drug type, administration route and administration period but may be selected adequately by those skilled in the art. To achieve the desired effect, the composition of the present disclosure may be administered at a dosage of 0.001-1000 mg/kg per day. The administration may be made once or several times a day. The administration dosage does not limit the scope of the present disclosure by any means.

Another aspect of the present disclosure relates to a pharmaceutical composition of the present invention for use according to the present invention wherein the treated subject is a non-human animal.

The administration of the pharmaceutical composition to a subject or cells may be achieved by injecting through a nasal, mucosal or inhalation route. The delivery method may be extended not only to cultured cells but also to general in-vivo delivery, i.e., delivery to animal cells, animal tissues and animal bodies.

Because the pharmaceutical composition is nonimmunogenic and non-communicable and the DNA is not packaged in a biological vector such as retrovirus or adenovirus, it is not restricted by the plasmid size. Accordingly, it can be used for a recombinant gene expression construct of any practical size.

### [Mode for Invention]

Hereinafter, the present disclosure will be described in detail through examples. However, the scope and contents of the present disclosure should not be interpreted to be reduced or limited by the following examples. In addition, it is obvious that those of ordinary skill in the art can easily carry out the present disclosure for which specific experimental data are not provided based on the teaching of the present disclosure including the examples and such change and modification belong to the scope of the appended claims.

### <Experimental methods>

### 1) Mice

8-week-old BALB/c mice were purchased from Orient Bio (Daejon, Korea). All the mice were housed in a specific pathogen-free animal facility. The mice were maintained on a 12-hour light-dark cycle with regular chow and autoclaved water.

German cockroach extract (hereinafter referred to as GCE)-induced asthma animal models were constructed under approval by the Animal Research Ethics Board of Yonsei University and intranasal protein delivery efficiency experiments were conducted under approval by the Animal Care and Use Committees and Ethics Board for Animal Research of Hanyang University.

### 2) German cockroach extract (GCE)

A German cockroach extract was prepared as follows. Pulverized German cockroach (*Blattella germanica*) was defatted in 200 mL of ether/ethyl acetate and slowly stirred at 4°C overnight in phosphate-buffered saline (PBS) with 6 mM β-mercaptoethanol and 1 mg/mL 1-phenyl-3-(2-thiazolyl)-2-thiourea. A German cockroach extract (hereinafter referred to as GCE) was obtained by filtering a supernatant obtained by centrifuging the mixture through a 0.22-µm filter after and then lyophilizing the same.

### 3) GCE-induced allergic asthma animal model

The mice reared according to the method of 1) were used. Specifically, the 8-week-old BALB/c mice were sensitized by intranasal administration of PBS (sham) or 120 µg of GCE prepared according to the method of 2) twice a week for 3 weeks.

To confirm the therapeutic effect of a fusion protein according to the present disclosure (dNP2-ctCTLA-4) in the GCE-induced chronic allergic asthma animal model, 10 µg of dNP2-ctCTLA-4 (Example 1), dNP2-EGFP (Comparative Example 1), CTLA4-Ig (Comparative Example 2) or PBS (control group) was intranasally administered together when the GCE was administered to the mice (see Test Example 2).

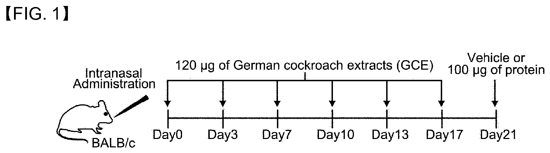

To confirm the intranasal delivery efficiency to the lungs, 100 µg of dTomato, Hph-1-dTomato, dNP2-dTomato or PBS was intranasally administered at day 21 for 15 minutes. The mice were sacrificed and the delivery efficiency of the proteins and the pathological changes in the lungs were analyzed (see FIGS. 1-3 and Test Example 1).

### 4) Airway hyperresponsiveness (AHR) analysis for methacholine

AHR was measured 4 days after the last GCE administration using a flexiVent 5.1 small-animal ventilator (SCIREQ, Montreal, PQ, Canada). The mice were placed in a whole body plethysmograph (Buxco, USA) and 0.4 mL of methacholine solutions at various concentrations (3.1, 6.25, 12.5, 25 or 50 mg/mL) in aerosols were administered through inhalation for 10 seconds. Penh was measured 1 minute and 2 minutes after the administration and the mean value was determined as the Penh (enhanced pause) value for the corresponding methacholine administration dosage.

### 5) Analysis of cell number and immune cell distribution in bronchoalveolar lavage fluid (BALF)

In order to collect the bronchoalveolar lavage fluid, the mouse lungs were washed with 1 mL of HBSS (Hank's balanced salt solution) with an intubated tube fluid. Total cells in the lavage fluid were counted with a hemocytometer. Then, the lavage fluid was centrifuged and the cells were prepared on slides after centrifugation at 1000 rpm for 3 minutes. The slides were stained with a Hemacolor staining kit (Merck Millipore, Darmstadt, Germany). The cells were differentially counted until the total counted number reached at least 200 according to the standard hemocytologic procedures to count macrophages and eosinophils.

### 6) Analysis of cytokine expression in bronchoalveolar lavage fluid

The lavage fluid (BALF) obtained by the method of 5) was homogenized using the T-PER tissue protein extraction reagent (Thermo Fisher Scientific, MA, US) to prepare a suspension. The suspension was incubated at 4 °C for 30 minutes and then centrifuged at 2500 rpm for 10 minutes. The supernatant was filtered with a 0.45-µm filter to analyze cytokine expression levels.

### 7) Observation of lung tissue

The lung was fixed in a 10% formalin solution for a day and tissues were prepared as paraffin blocks and sectioned to 3- to 4-µm thickness using a microtome. The prepared slides were stained with PAS (periodic acid-Schiff) stain or Masson's trichrome stain. The stained tissues were observed with a microscope (Olympus BX40).

### 8) Th2 differentiation assay

Mouse naive CD4 T cells were isolated from the 8-week-old BALB/c mice by magnetic-activated cell sorting (MACS, naive CD4 T cell isolation kit, mouse, Miltenyi, Bergisch Gladbach, Germany).

Then, 2.5x10⁵ of the isolated cells were seeded onto anti-CD3/anti-CD28 antibody (0.1 µg/mL)-coated 96-well plates. The cells were incubated in the presence of anti-IFN-γ neutralizing antibody (5 µg/mL), IL-2 (50 U/mL), IL-4 (30 ng/mL) and PBS. After adding 1 µM dNP2-EGFP (Comparative Example 3) or 1 µM dNP2-ctCTLA-4 (Example 2) to each well for 6 days, the cells were analyzed by flow cytometry after re-stimulation, protein transport inhibitor treatment and intracellular staining with anti-mouse IFN-γ-FITC and anti-mouse IL-4-PE FACS antibodies.

### 9) Statistical analysis

All data were statistically analyzed by one-way analysis of variance (one-way ANOVA) using the Prism 6 software (GraphPad). P-values < 0.05 were considered statistically significant.

### <Preparation Example 1> Synthesis and purification of dNP2, ctCTLA-4 and Hph-1 peptides

Peptides having amino acid sequences of SEQ ID NOS 1-3 were synthesized.

The cell-penetrating peptide with an amino acid sequence of SEQ ID NO 1 was named as 'dNP2', the peptide with an amino acid sequence of SEQ ID NO 2 was named as 'ctCTLA-4' and the cell-penetrating peptide with an amino acid sequence of SEQ ID NO 3 was named as 'Hph-1'.

After synthesizing sense and antisense oligodeoxynucleotides for the amino acid sequences, they were kept at 95 °C for 3 minutes to remove any secondary or tertiary structures formed (denaturation). Then, double-stranded DNAs were prepared by changing temperature to 50 °C and then to 72 °C. For insertion to the pRSET-b vector, restriction enzyme-specific sequences inserted to 5' and 3' ends in addition to the sense and antisense oligodeoxynucleotides. Then, *Escherichia coli* BL21(DE3) Star pLysS was transformed with the pRSET-b plasmid encoding each peptide. The colony was cultured in ampicillin-containing Luria-Bertani medium at 37°C and protein expression was induced by 1 mM isopropyl-β-D-thiogalactopyranoside (IPTG). The proteins were purified by Ni-NTA affinity chromatography (Qiagen) and desalted using a PD-10 column (GE Healthcare). All the proteins were stored at -80 °C.
SEQ ID NO 1
   KIKKVKKKGRKGSKIKKVKKKGRK
SEQ ID NO 2 (ctCTLA-4)
   KMLKKRSPLTTGVYVKMPPTEPECEKQFQPYFIPIN
SEQ ID NO 3 (Hph-1)
   YARVRRRGPRR

### <Example 1> Preparation of dNP2-ctCTLA-4 fusion protein

In order to fuse the cell-penetrating peptide having an amino acid sequence of SEQ ID NO 1 and the ctCTLA-4 peptide having an amino acid sequence of SEQ ID NO 2 prepared in Preparation Example 1, a primer allowing the cell-penetrating peptide represented by SEQ ID NO 1 to be linked to the N-terminal of the ctCTLA-4 peptide was prepared. After producing the dNP2-ctCTLA-4 gene through PCR reaction, it was inserted into a vector (pRSET-b). After purifying the protein expressed in E. *coli,* intracellular delivery efficiency was investigated. Detailed experimental procedures are described below.

### 1) Preparation of encoding genes

A forward primer was prepared by inserting a DNA base sequence encoding the cell-penetrating peptide having an amino acid sequence of SEQ ID NO 1 to a DNA base sequence encoding a part of the N-terminal of the ctCTLA-4 peptide having an amino acid sequence of SEQ ID NO 2 prepared in Preparation Example 1. The SEQ ID NO and restriction enzyme recognition site of each primer are summarized in Table 1.

PCR reaction was conducted using the primer of SEQ ID NO 4 with the pRSETb vector containing the gene encoding the peptide of SEQ ID NO 2 as a template.

After initial thermal denaturation at 95 °C for 3 minutes, 30 cycles of thermal denaturation of the template at 95 °C for 20 seconds, polymerization of the primer with the template at 50 °C for 20 seconds and extension at 72 °C for 30 seconds were conducted using a PCR reactor (Bio-Rad).

**[Table 1]**

| No. | Primers | Base sequences |
|---|---|---|
| SEQ ID NO 4 | 1st forward primer of dNP2-ctCTLA-4 | AAGATTAAGAAAGTCAAGAAGAAAGGAAGAAAGG AATTCTACCCATACGATGTTCCAGATTACGCTA |
| SEQ ID NO 5 | 2nd forward primer of dNP2-ctCTLA-4 | GCTAGCAAGATTAAGAAAGTCAAGAAGAAAGGAA GAAAGGGATCCAAGATTAAGAAAGTCAAGAAGA |

For the dNP2-ctCTLA-4 forward primer, PCR was conducted in two stages because the sequence of dNP2 (KIKKVKKKGRKGSKIKKVKKKGRK) was too long.

### 2) Preparation of recombinant expression vector

In order to express the dNP2-ctCTLA-4 fusion protein, the gene (DNA) fragment prepared in 1) of Example 1 was cleaved using a restriction enzyme and then inserted into the protein expression vector pRSETb using a ligase.

The DNA fragment amplified in 1) of Example 1 was treated with Nhel and HindIII (NEB) enzymes to make the 5'- and 3'-ends of the DNA sticky. Meanwhile, pRSETb was treated with the same restriction enzymes to prepare a linear pRSETb vector having Nhel and HindIII insertion sites. After each enzymatic reaction, the product was separated using a PCR purification kit (Cosmo Genetech).

The separated dNP2-ctCTLA-4 double-stranded DNA fragment and the pRSET-b vector were treated with T4 ligase (NEB) at 25 °C for 2 hours.

The resulting circular pRSETb vector in which dNP2-ctCTLA-4 was inserted was transformed into DH5α *E*. *coli.* The transformed E. *coli* which formed a colony when cultured on a plate LB medium containing 50 µg/mL ampicillin as an antibiotic was selected. The selected *E*. *coli* colony was cultured again in a liquid LB medium containing 50 µg/mL ampicillin and then the plasmid vector was separated using a plasmid minipreparation kit (Cosmo Genetech).

In order to confirm that the separated plasmid vector is a pRSETb vector in which dNP2-ctCTLA-4 is inserted, it was treated with Nhel and HindIII restriction enzymes and then analyzed by DNA base sequencing (Bionics).

### 3) Isolation and purification of protein

The pRSETb vector in which dNP2-ctCTLA-4 is inserted, prepared in 2) of Example 1, was transformed into *E*. *coli* BL21 (DE3) star pLysS. A colony formed on a plate LB medium containing 34 µg/mL chloramphenicol and 50 µg/mL ampicillin as antibiotics was cultured in 50 mL of a liquid LB medium at 37 °C for 10 hours and then transferred to 500 mL of a fresh liquid LB medium. After culturing at the same temperature until the quantity of *E*. *coli* measured by a spectrophotometer reached O.D. 0.5, IPTG (isopropyl β-D-1-thiogalactopyranoside) was added to a concentration of 1 mM and the *E*. *coli* was further cultured in a shaking incubator set to 20 °C and 150 rpm for 14 hours. The protein expressed by the *E*. *coli* contained a 6X-His tag upstream of the pRSET-b vector. The protein was purified as follows.

The culture was centrifuged and then resuspended in a lysis buffer (0.5 M NaCl, 5 mM imidazole, 20 mM Tris-HCl, pH 8.0) under a native condition. The *E*. *coli* was allowed to be suspended in the lysis buffer for 10 minutes in order to disrupt the cell wall and cell membrane. Then, the cells were disrupted using the ultrasonic cell crusher VCX-130 (Sonics & Materials) and then centrifuged. The separated supernatant was filtered once through a 0.45-µm filter (Advantec) and was allowed to bind to Ni-NTA agarose (Qiagen) at room temperature for 1 hour. Then, only the protein product binding to the Ni-NTA agarose was made to bind to a histidine column (His-column, Bio-Rad). After washing with 20 mM and 250 mM imidazole solutions, the protein was eluted using a 3 M imidazole solution. Finally, the dNP2-ctCTLA-4 fusion protein was purified from the eluted protein product using a PD-10 desalination column (Amersham Biosciences).

### <Comparative Example 1> Synthesis of dTomato

The red fluorescent protein dTomato was used.

### <Comparative Example 2> Synthesis of Hph-1-dTomato

A fusion protein in which the cell-penetrating peptide of SEQ ID NO 3 prepared in Preparation Example 1 is fused with the red fluorescent protein dTomato was synthesized by Cosmo Genetech.

### <Comparative Example 3> Synthesis and purification of dNP2-EGFP

In order to fuse the cell-penetrating peptide of SEQ ID NO 1 prepared in Preparation Example 1 with an enhanced green fluorescent protein (EGFP), a primer was constructed which allows linking of EGFP at the N-terminal of dNP2. After producing a dNP2-EGFP gene through PCR, it was inserted into a vector (pRSET-b). The protein was expressed in *E*. *coli* and then purified in the same manner as in Example 1 except for the primers. The following primers were used.
[SEQ ID NO 6] (1st forward primer) 
AAGATTAAGAAAGTCAAGAAGAAAGGAAGAAAGGTGAGCAAGGGCGAG
GAGCTGTTCACCG
[SEQ ID NO 7] (2nd forward primer)

GCTAGCAAGATTAAGAAAGTCAAGAAGAAAGGAAGAAAGGGATCCAAGA
TTAAGAAAGTCAAGAAGA

### <Comparative Example 4> ctCTLA-4-Ig

Abatacept, which is a recombinant protein consisting of the CTLA-4 extracellular domain conjugated with the immunoglobulin G constant area (CTLA-4-Ig) and known as a therapeutic agent for rheumatoid arthritis, was used.

### <Test Example 1> Protein delivery efficiency of dNP2 peptide into lung resident cells via intranasal administration

### 1) Design of experiments

Because the dNP2 peptide can more efficiently deliver a protein to activated or memory-like immune cells than it can to resting cells, it was hypothesized that the dNP2 peptide could deliver a protein to the inflammatory lung resident cells more effectively than it could in sham mice treated with nothing. The specific experimental conditions are shown in FIG. 1.

### <Control groups>

PBS was intranasally administered to a non-treated animal model (8-week-old female BALB/c mice, see 1) of Experimental methods) twice (120 µg per each) a week for 3 weeks. On day 21, 100 µg of a vehicle (PBS) or protein (dTomato, Hph-1-dTomato or dNP2-dTomato) was administered through the nasal cavity.

Depending on the vehicle or proteins administered on day 21, the control groups were denoted as a dTomato control group, an Hph-1-d Tomato control group, a dNP2-dTomato control group or a PBS control group.

### <Test groups>

In order to induce chronic allergic inflammation in the lungs of the animal model, GCE was intranasally administered to 8-week-old female BALB/c mice twice (120 µg per each) a week for 3 weeks (see 3) of Experimental methods). On day 21, 100 µg a vehicle (PBS) or protein (dTomato, Hph-1-dTomato or dNP2-dTomato) was administered through the nasal cavity.

Depending on the vehicle or proteins administered on day 21, the test groups were denoted as a dTomato test group, an Hph-1-d Tomato test group, a dNP2-dTomato test group or a PBS test group.

### 2) Results

15 minutes after the experiment was completed, a bronchoalveolar lavage fluid (BALF) was obtained by injecting 1 mL of PBS twice to each animal. After euthanasia, the lung tissues were harvested, washed with PBS and fixed with 4% paraformaldehyde. All the tissues were frozen using the O.C.T. compound (Wako Chemical). The frozen blocks were sectioned to 8 µm thickness using a cryostat (Thermo Scientific).

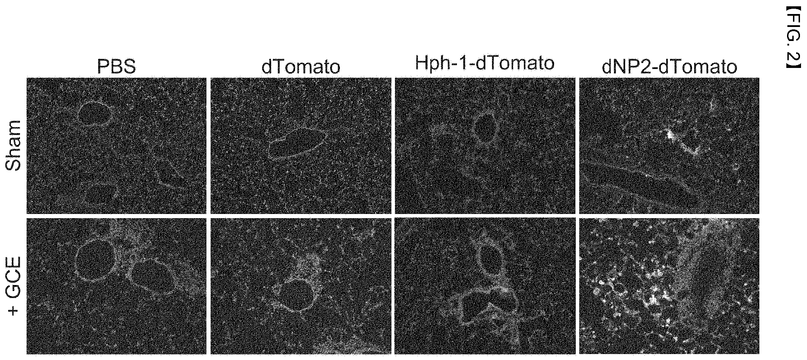

FIG. 2 shows result of preparing frozen lung tissue sections (8 µm) from each control group and test group of Test Example 1, staining cell nuclei with Hoechst and observing by fluorescence microscopy.

As seen from FIG. 2, when protein signals from the lung tissue of each animal model (sham mouse or GCE-induced allergic asthma animal model) were observed 15 minutes after the administration of the vehicle or protein on day 21, dNP2-dTomato was observed in the airway epithelium and some resident cells. However, for the Hph-1-dTomato control group and the dTomato control group showed comparable levels with the PBS control group, suggesting that the protein delivery efficiency of the dNP2 peptide is the most superior in the non-treated animal model (sham mouse).

The results for the test groups showed similar tendency to that of the control groups. But, the dNP2-dTomato test group showed the brightest signals as compared to the dTomato test group, the Hph-1-d Tomato test group and the PBS test group in the inflammatory lung resident cells.

As expected, the dNP2 peptide showed superior protein delivery efficiency for the lung resident cells in both the sham mouse to which PBS was administered for a period of long time and the GCE-induced allergic asthma animal model.

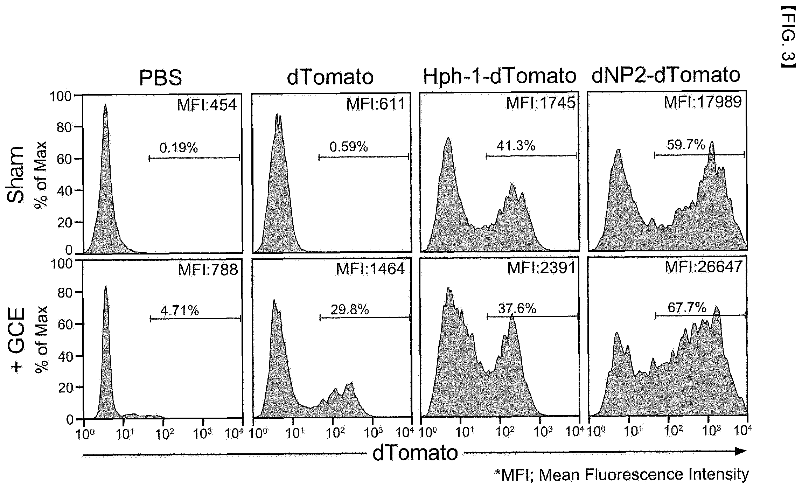

FIG. 3 shows a flow cytometry result of fluorescence signals from the bronchoalveolar lavage fluid of each control group and test group of Test Example 1. The red fluorescence histograms and mean fluorescence intensities (MFI) are shown.

As seen from FIG. 3, when the bronchoalveolar lavage fluid (BALF) recovered from each animal was analyzed by flow cytometry, the dNP2-dTomato test group and control group showed 10 times higher mean fluorescence intensity (MFI) than the Hph-1-dTomato test group and control group. This suggest that the dNP2 peptide shows much higher protein delivery efficiency to the lung cells via intranasal administration as compared to the Hph-1 peptide.

### <Test Example 2> Analysis of therapeutic effect of dNP2-ctCTLA-4 fusion protein in GCE-induced allergic asthma animal model

### 1) Design of experiments

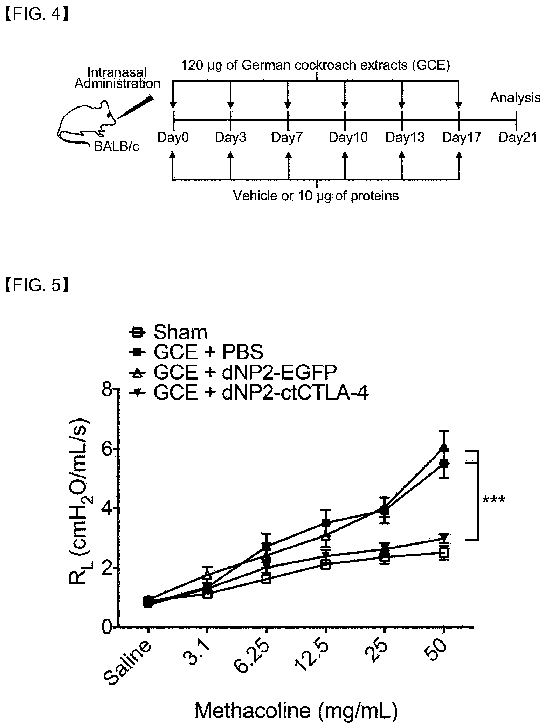

Next, experiments were designed as follows to determine the therapeutic effect of the fusion protein according to the present disclosure (dNP2-ctCTLA-4) in a GCE-induced allergic asthma animal model (see 3) of Experimental methods). The specific experimental conditions are shown in FIG. 4.

The mice reared according to 1) of Experimental methods were used. Specifically, PBS (sham) or 120 µg of GCE prepared according to 2) of Experimental methods was intranasally administered to the 8-week-old BALB/c mice twice a week for 3 weeks. 10 µg of dNP2-ctCTLA-4 (Example 1), dNP2-EGFP (Comparative Example 3), CTLA4-Ig (Comparative Example 2) or a vehicle (PBS) was intranasally administered together when the GCE was administered to the mice. On day 21, the mice were anesthetized and hyperresponsiveness to methacholine (MeCh) was measured (see 4) of Experimental methods).

### <Control group>

PBS was intranasally administered to the 8-week-old female BALB/c mice reared according to 1) of Experimental methods twice (120 µg per each) a week for 3 weeks. 10 µg of a vehicle (PBS) was intranasally administered together when the PBS was administered to the mice. This group was denoted as a sham control group.

### <Test groups>

The mice reared according to 1) of Experimental methods were used. Specifically, 120 µg of GCE prepared according to 2) of Experimental methods was intranasally administered to the 8-week-old BALB/c mice twice a week for 3 weeks. 10 µg of dNP2-ctCTLA-4 (Example 2), dNP2-EGFP (Comparative Example 3) or a vehicle (PBS) was intranasally administered together when the GCE was administered to the mice.

Depending on the administered proteins or vehicle, the test groups were denoted as a dNP2-ctCTLA-4 test group, a dNP2-EGFP test group or a PBS test group.

### 2) Results

On day 21, the animals were anesthetized and hyperresponsiveness to methacholine (MeCh) was measured (see 4) of Experimental methods).

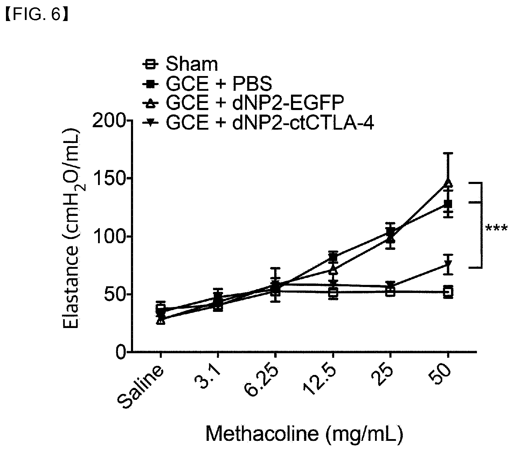

FIG. 5 and FIG. 6 show the result of analyzing the airway hyperresponsiveness (AHR) to methacholine for each test group and control group of Test Example 2. FIG. 5 shows the airway resistance of each test group and control group of Test Example 2 measured depending on the methacholine concentration. FIG. 6 shows the airway elastance of each test group and control group of Test Example 2 measured depending on the methacholine concentration. n = 5 per group and the results are presented as mean ± s.e.m. (standard error of mean). **p < 0.01, ***p < 0.001.

As seen from FIG. 5 and FIG. 6, the PBS test group and the dNP2-EGFP test group showed significantly increased airway resistance and airway elastance, indicating significant airway hyperresponsiveness owing to the reduced airway space. The dNP2-ctCTLA-4 test group treated with 10 µg of dNP2-ctCTLA-4 showed significantly reduced airway hyperresponsiveness, suggesting that the airway hyperresponsiveness induced by multiple exposures to GCE was ameliorated by the intranasal administration of dNP2-ctCTLA-4. That is to say, it was confirmed that dNP2-ctCTLA-4 has a therapeutic or preventive effect for GCE-induced allergic asthma.

### <Test Example 3> Airway inflammation improving effect of dNP2-ctCTLA-4 fusion protein in GCE-induced allergic asthma animal model

To evaluate the inhibitory effect of the dNP2-ctCTLA-4 fusion protein on the pathophysiology of lungs in the GCE-induced allergic asthma animal model, the lung tissues of each test group and control group of Test Example 2 was observed according to 7) of Experimental methods.

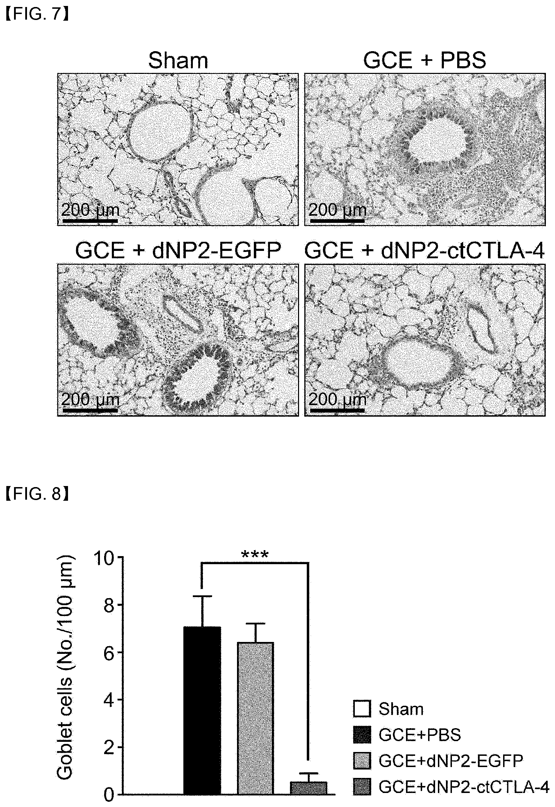

FIG. 7 shows the result of observing lung tissues for each test group and control group of Test Example 3. The lung tissue of each test group and control group of Test Example 3 was imaged after staining with PAS. The red region indicates mucus-secreting goblet cells. FIG. 8 shows a result of analyzing the number of the mucus-secreting goblet cells in FIG. 7 using the ImageJ 1.50i software.

As seen from FIG. 7 and FIG. 8, the intranasal GCE administration substantially increased infiltrated mononuclear cells around blood vessels as well as goblet cell metaplasia in the airway epithelium. However, the intranasal administration of the dNP2-ctCTLA-4 fusion protein according to the present disclosure significantly reduced inflammatory cells and goblet cells in the lungs.

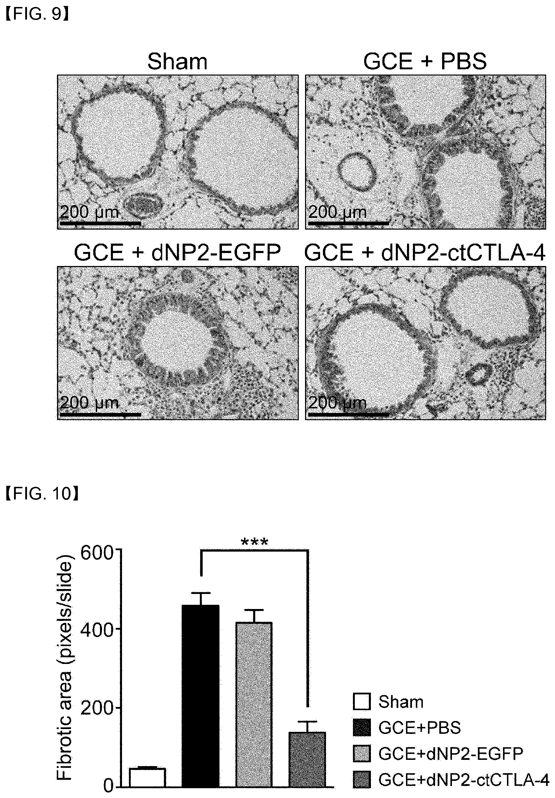

FIG. 9 shows a result of observing the lung tissues for each test group and control group of Test Example 3. The lung tissues of each test group and control group of Test Example 3 were imaged after staining with Masson's trichrome stain. The blue region indicates collagen deposition and fibrosis.

FIG. 10 shows a result of analyzing the fibrotic area of FIG. 9 using the ImageJ 1.50i software. The representative results for each group (n = 5) are presented as mean ± s.e.m. (standard error of mean). ***p < 0.001.

In FIG. 9 and FIG. 10, the fibrotic area was measured by staining each test group and control group with Masson's trichrome stain. A high level of peribronchial fibrosis in the lung was observed for the test group (PBS test group) as compared to the control group (sham). Meanwhile, the dNP2-ctCTLA-4 test group intranasally administered with the dNP2-ctCTLA-4 fusion protein showed significantly reduced fibrotic area around the bronchial regions.

From these results, it was confirmed that the intranasal administration of the dNP2-ctCTLA-4 fusion protein according to the present disclosure significantly improves airway inflammation and fibrosis.

### <Test Example 4> Inhibitory effect of eosinophil infiltration and Th2 cytokine production of dNP2-ctCTLA-4 fusion protein in GCE-induced allergic asthma animal model

### 1) Design of experiments

Experimental conditions were designed as in Test Example 2. A bronchoalveolar lavage fluid was obtained from each test group and control group of Test Example 2 and the inhibitory effect of eosinophil infiltration and Th2 cytokine production was investigated. The details of the design of experiments are as follows.

### <Control group>

PBS was intranasally administered to the 8-week-old female BALB/c mice reared according to 1) of Experimental methods twice (120 µg per each) a week for 3 weeks. 10 µg of a vehicle (PBS) was intranasally administered together when the PBS was administered to the mice. This group was denoted as a sham control group.

### <Test groups>

The mice reared according to 1) of Experimental methods were used. Specifically, 120 µg of GCE prepared according to 2) of Experimental methods was intranasally administered to the 8-week-old BALB/c mice twice a week for 3 weeks. 10 µg of dNP2-ctCTLA-4 (Example 2), dNP2-EGFP (Comparative Example 3) or a vehicle (PBS) was intranasally administered together when the GCE was administered to the mice.

Depending on the administered proteins or vehicle, the test groups were denoted as a dNP2-ctCTLA-4 test group, a dNP2-EGFP test group or a PBS test group.

### 2) Results

15 minutes after the experiment was completed, a bronchoalveolar lavage fluid (BALF) was obtained by injecting 1 mL of PBS twice to each animal (see 5) of Experimental methods).

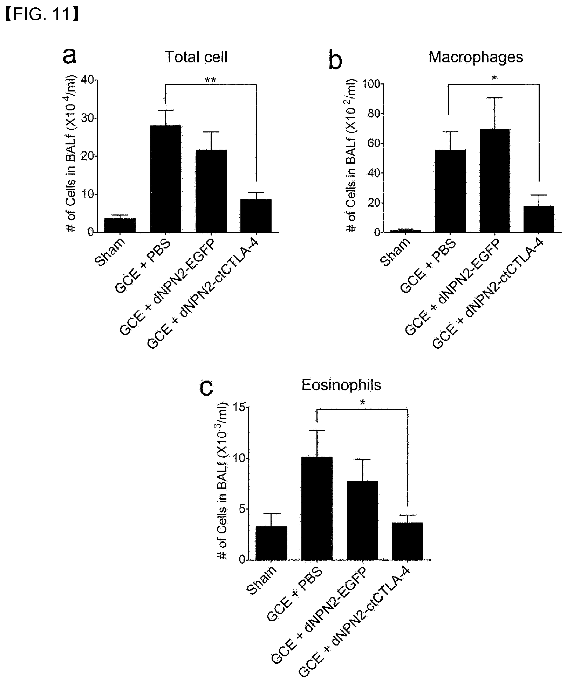

FIG. 11 shows a result of cytocentrifuging the bronchoalveolar lavage fluid recovered from each test group and control group of Test Example 4 on a slide and counting the number of total cells (a), macrophages (b) and eosinophils (c) after staining using the Hemacolor staining kit.

As seen from FIG. 11, the GCE-administered PBS test group showed increased number of total cells (a), macrophages (b) and eosinophils (c) in the bronchoalveolar lavage (BAL) as compared to the control group (sham).

In contrast, the dNP2-ctCTLA-4 test group to which the dNP2-ctCTLA-4 fusion protein was intranasally administered together with GCE showed 3 times or more decrease in the number of total cells (a), macrophages (b) and eosinophils (c) in the bronchoalveolar lavage (BAL). Accordingly, it was confirmed that the treatment with the dNP2-ctCTLA-4 fusion protein according to the present disclosure provides therapeutic and preventive effect in the GCE-induced allergic asthma animal model.

FIG. 12 shows a result of analyzing the expression level of IL-5, IL-13 and IFN-γ in a supernatant of a bronchoalveolar lavage (BAL) using the ELISA kit (top) and a result of analyzing the expression level of IL-5, IL-13, IL-4 and IFN-γ in the frozen lung tissues using the ELISA kit (bottom). The concentration of IL-5, IL-13, IL-4 and IFN-γ was analyzed with the ELSISA kit and the result was normalized to the total protein concentration of the tissue. The protein concentration was quantified by the Bradford assay. n = 5 per each group and the results are presented as mean ± s.e.m. (standard error of mean). *p < 0.05, **p < 0.01.

In this experiment, the bronchoalveolar lavage (BAL) and lung tissues were recovered from each test group and control group and the expression level of IL-13, IL-4 and Th2 cytokines was compared.

As seen from FIG. 12, the GCE-administered PBS test group showed significantly increased expression of IL-5, IL-13, IL-4 and IFN-γ in the bronchoalveolar lavage (BAL) and lung tissues as compared to the control group (sham).

However, the dNP2-ctCTLA-4 test group treated with the dNP2-ctCTLA-4 fusion protein according to the present disclosure showed more than 3 times and 2 times decreased expression levels of IL-5, IL-13, IL-4 and IFN-γ both in the bronchoalveolar lavage (BAL) and lung tissues. Because IL-5 is critical for eosinophil activation, it can be seen that the dNP2-ctCTLA-4 fusion protein according to the present disclosure inhibits eosinophil activation.

It was also found out that the dNP2-ctCTLA-4 test group treated with the dNP2-ctCTLA-4 fusion protein showed decreased level of IFN-γ, suggesting that the intranasal administration of dNP2-ctCTLA-4 significantly reduces Th2 inflammation which is associated with eosinophil recruitment and cytokine production.

### <Test Example 5> Inhibitory effect of airway inflammation of dNP2-ctCTLA-4 fusion protein in GCE-induced allergic asthma animal model

### 1) Design of experiments

Abatacept is a recombinant protein composed of the extracellular domain of CTLA-4 and the Fc region of immunoglobulin (CTLA4-Ig). It is the most widely known immune regulatory drug at present. It is known that systemic administration of CTLA4-Ig can inhibit T-cell activation and airway inflammation in experimental asthma models

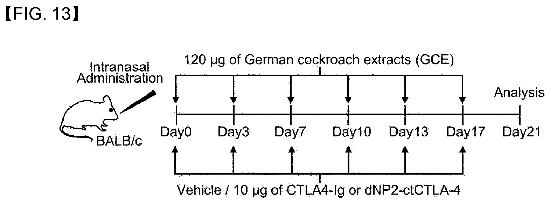

However, whereas the dNP2-ctCTLA-4 fusion protein according to the present disclosure can achieve remarkably superior lung cell permeability and therapeutic and preventive effect for airway inflammation through intranasal administration, CTLA4-Ig cannot inhibit airway inflammation when injected through the intranasal route due to low tissue permeability. This was demonstrated through experiments as follows. The experimental conditions are shown in FIG. 13.

### <Control group>

PBS was intranasally administered to the 8-week-old female BALB/c mice reared according to 1) of Experimental methods twice (120 µg per each) a week for 3 weeks. 10 µg of a vehicle (PBS) was intranasally administered together when the PBS was administered to the mice. This group was denoted as a sham control group.

### <Test groups>

The mice reared according to 1) of Experimental methods were used. Specifically, 120 µg of GCE prepared according to 2) of Experimental methods was intranasally administered to the 8-week-old BALB/c mice twice a week for 3 weeks. 10 µg of dNP2-ctCTLA-4 (Example 2), CTLA4-lg (Comparative Example 4) or a vehicle (PBS) was intranasally administered together when the GCE was administered to the mice.

Depending on the administered proteins or vehicle, the test groups were denoted as a dNP2-ctCTLA-4 test group, a CTLA4-Ig test group or a PBS test group.

### 2) Results

15 minutes after the experiment was completed, a bronchoalveolar lavage fluid (BALF) was obtained by injecting 1 mL of PBS twice to each animal (see 5) of Experimental methods).

FIG. 14 shows a result of analyzing the expression level IL-13 and IFN-γ in a supernatant of a bronchoalveolar lavage (BAL) using the ELISA kit (top) and a result of analyzing the expression level of IL-13, IL-4 and IFN-γ in the frozen lung tissue using the ELISA kit (bottom). The concentration of IL-13, IL-4 and IFN-γ was analyzed with the ELSISA kit and the result was normalized to the total protein concentration of the tissue. The protein concentration was quantified by the Bradford assay. n = 5 per each group and the results are presented as mean ± s.e.m. (standard error of mean). *p < 0.05, **p < 0.01.

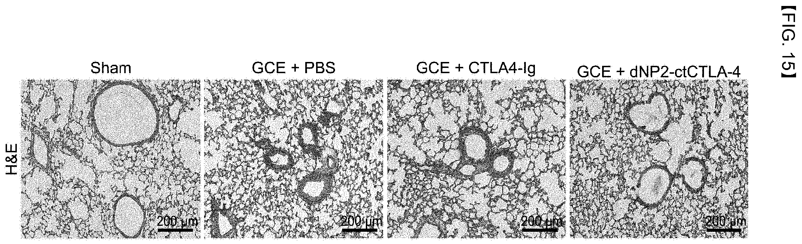

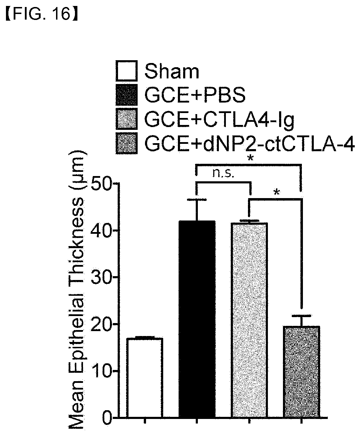

As seen from FIG. 14, the dNP2-ctCTLA-4 test group intranasally administered with the dNP2-ctCTLA-4 fusion protein according to the present disclosure showed significantly decreased levels of IL-13 and IFN-γ in the bronchoalveolar lavage (BAL) and lung tissues. However, the CTLA4-Ig test group intranasally administered with CTLA4-Ig showed 3 times higher cytokine production as compared to the control group (sham), suggesting that CTLA4-Ig cannot effectively inhibit airway inflammation when administered through the nasal cavity. FIG. 15 shows microscopic images of the lung tissues recovered from each test group and control group of Test Example 5 imaged after staining with H&E (hematoxylin and eosin). The representative images for each group (n = 5) are shown. FIG. 16 shows a result of measuring the airway epithelial thickness of FIG. 15 using the ImageJ 1.50i software. The result is presented as mean ± s.e.m. (standard error of mean). *p < 0.05, **p < 0.01.

Referring to FIG. 15 and FIG. 16 which were obtained by staining the lung tissues obtained from each test group and control group with hematoxylin eosin (H&E), it can be seen that the dNP2-ctCTLA-4 test group intranasally administered with dNP2-ctCTLA-4 showed improved physiological features of the respiratory epithelium, whereas the CTLA4-Ig test group showed no inhibition of cell infiltration and airway epithelium thickening. From these results, it was confirmed that the intranasal administration of the dNP2-ctCTLA-4 fusion protein according to the present disclosure provides 2-3 times better pharmacological effect in the GCE-induced allergic asthma animal model than the existing CTLA4-Ig.

### <Test Example 6> Inhibitory effect of Th2 cell differentiation of dNP2-ctCTLA-4 fusion protein in GCE-induced allergic asthma animal model

It was investigated whether the dNP2-ctCTLA-4 fusion protein can directly inhibit the differentiation of Th2 cells in the GCE-induced allergic asthma animal model. The experiment was conducted according to 8) of Experimental methods.

MACS-sorted CD4⁺ CD62L^{hi}CD44⁻ native CD4 T cells isolated from 8-week-old BALB/c mice were used. They were differentiated into Th2 cells by addition of IL-4 during activation with anti-CD3/CD28 antibodies for 6 days.

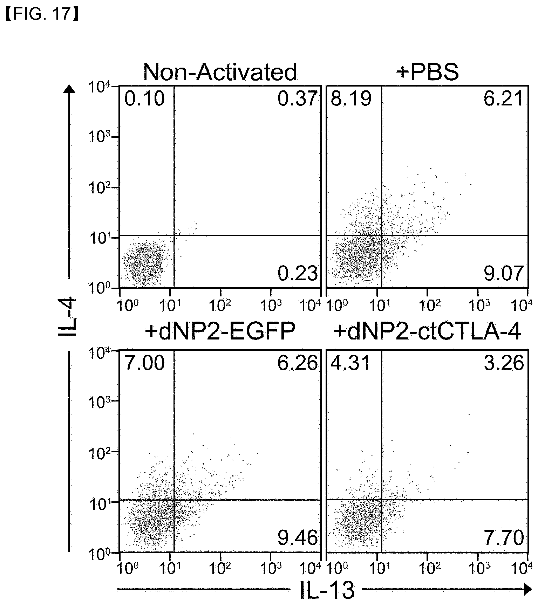

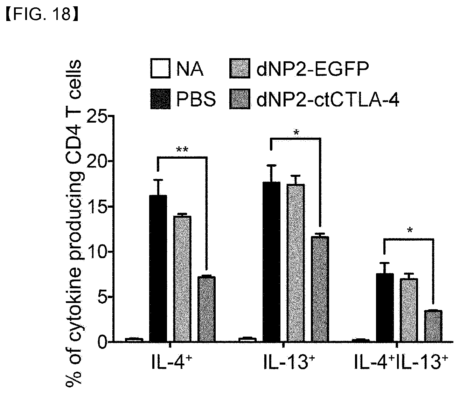

FIG. 17 shows a result of analyzing the differentiation of Th2 cells by non-activated, +PBS, +dNP2-EGFP and +dNP2-ctCTLA-4. FIG. 18 shows a result of calculating the percentage (%) of cytokines produced in CD4⁺IL-4⁺ differentiated Th2 cells. The result is presented as mean ± s.e.m. (standard error of mean) (n = 3). **p < 0.01. NA (non-activated) denotes a negative control group, not stimulated by cytokines or anti-TcR antibody.

As seen from FIGS. 17 and 18, the Th2 cells were analyzed to produce IL-4 and IL-13 by flow cytometry. The differentiated Th2 cells had a significant proportion of IL-4 (and/or IL-13)-producing CD4 T cells, while the dNP2-ctCTLA-4 group showed significantly reduced cytokine production, demonstrating that the differentiation of Th2 cells is inhibited by dNP2-ctCTLA-4. These results suggest that the dNP2-ctCTLA-4 fusion protein according to the present disclosure directly inhibits Th2 cell differentiation, contributing to its inhibitory effect of Th2 inflammation in the lungs of the GCE-induced animal model.

### [Industrial Applicability]

The fusion protein in which a cell-penetrating peptide and a ctCTLA4 peptide are fused of the present disclosure can provide quick, fast, and effective therapeutic or preventive effect for inflammatory respiratory diseases, particularly allergic asthma, via administration through the respiratory system, with which effective therapeutic or preventive effect could not be achieved, by providing superior delivery and targeting efficiency for a bronchus and lung cells and remarkably improved activities of suppressing cytokine expression, suppressing airway inflammation, alleviating airway hyperresponsiveness to allergens, alleviating Th2 inflammation, etc. as compared to the existing therapeutic agents.

## Claims

1. A pharmaceutical composition for use through intranasal administration in preventing or treating an inflammatory respiratory disease, comprising as an active ingredient a protein in which a cell-penetrating peptide dNP2 of SEQ ID NO 1 (KIKKVKKKGRKGSKIKKVKKKGRK) and a ctCTLA4 peptide of SEQ ID NO 2 (KMLKKRSPLTTGVYVKMPPTEPECEKQFQPYFIPIN) are fused.

2. The pharmaceutical composition for use according to claim 1, wherein the pharmaceutical composition is administered by being inhaled in the form of a spray or a powder.

3. The pharmaceutical composition for use according to claim 1, wherein the pharmaceutical composition has permeation activity for the nasal mucosa, bronchial mucosa or lung epithelial cells.

4. The pharmaceutical composition for use according to claim 1, wherein the inflammatory respiratory disease is selected from a group consisting of asthma, chronic obstructive pulmonary disease (COPD), acute lung injury, pyothorax, lung abscess, pneumonia, pulmonary tuberculosis, bronchitis, sore throat, tonsillitis, paranasal sinusitis, rhinitis, constrictive bronchiolitis and laryngitis.

5. The pharmaceutical composition for use according to claim 4, wherein the asthma is an allergic asthma caused by dust mite, pollen, animal fur or dander, cockroach, food, drug, flu, cigarette smoke, indoor contamination, air pollution, food additive, exercise, climate change, yellow dust or stress.

6. The pharmaceutical composition for use according to claim 5, wherein the asthma is an allergic asthma caused by cockroach.

7. The pharmaceutical composition of claim 1 for use according to claim 1 wherein the treated subject is a non-human animal.

## Patentansprüche

1. Pharmazeutische Zusammensetzung zur Verwendung durch intranasale Verabreichung bei der Prävention oder Behandlung einer entzündlichen Atemwegserkrankung, die als Wirkstoff ein Protein umfasst, bei dem ein zellpenetrierendes Peptid dNP2 der SEQ ID Nr. 1 (KIKKVKKKGRKGSKIKKVKKKGRK) und ein ctCTLA4-Peptide der SEQ ID Nr. 2 (KMLKKRSPLTTGVYVKMPPTEPECEKQFQPYFIPIN) miteinander fusioniert sind.

2. Pharmazeutische Zusammensetzung zur Verwendung gemäß Anspruch 1, wobei die pharmazeutische Zusammensetzung dadurch verabreicht wird, dass sie in Form eines Sprays oder eines Pulvers inhaliert wird.

3. Pharmazeutische Zusammensetzung zur Verwendung gemäß Anspruch 1, wobei die pharmazeutische Zusammensetzung eine Permeationsaktivität für die Nasenschleimhaut, Bronchialschleimhaut oder Lungenepithelzellen aufweist.

4. Pharmazeutische Zusammensetzung zur Verwendung gemäß Anspruch 1, wobei die entzündliche Atemwegserkrankung aus der Gruppe ausgewählt ist, die aus Asthma, chronisch obstruktiver Lungenerkrankung (COPD), akuter Lungenverletzung, Pyothorax, Lungenabszess, Lungenentzündung, Lungentuberkulose, Bronchitis, Halsschmerzen, Tonsillitis, paranasaler Sinusitis, Rhinitis, Bronchiolitis obliterans und Laryngitis besteht.

5. Pharmazeutische Zusammensetzung zur Verwendung gemäß Anspruch 4, wobei das Asthma ein allergisches Asthma ist, welches durch Hausstaubmilben, Pollen, Tierhaare oder -schuppen, Schaben, Lebensmittel, Arzneistoffe, Grippe, Zigarettenrauch, häusliche Kontamination, Luftverschmutzung, Lebensmittelzusatzstoffe, sportliche Betätigung, Klimawandel, gelben Staub oder Stress verursacht wird.

6. Pharmazeutische Zusammensetzung zur Verwendung gemäß Anspruch 5, wobei das Asthma ein allergisches Asthma ist, das durch Schaben verursacht wird.

7. Pharmazeutische Zusammensetzung gemäß Anspruch 1 zur Verwendung gemäß Anspruch 1, wobei der behandelte Patient ein nichthumanes Tier ist.

## Revendications

1. Composition pharmaceutique à utiliser par administration intranasale pour prévenir ou traiter une maladie respiratoire inflammatoire, comprenant comme principe actif une protéine dans laquelle un peptide pénétrant les cellules dNP2 de SEQ ID n° 1 (KIKKVKKKGRKGSKIKKVKKKGRK) et un peptide ctCTLA4 de SEQ ID n° 2 (KMLKKRSPLTTGVYVKMPPTEPECEKQFQPYFIPIN) sont fusionnés.

2. Composition pharmaceutique à utiliser selon la revendication 1, dans laquelle la composition pharmaceutique est administrée par inhalation sous forme de spray ou de poudre.

3. Composition pharmaceutique à utiliser selon la revendication 1, dans laquelle la composition pharmaceutique a une activité de perméation pour la muqueuse nasale, la muqueuse bronchique ou les cellules épithéliales pulmonaires.

4. Composition pharmaceutique à utiliser selon la revendication 1, dans laquelle ladite maladie respiratoire inflammatoire est choisie dans le groupe consistant en asthme, bronchopneumopathie chronique obstructive (BPCO), lésion pulmonaire aiguë, pyothorax, abcès pulmonaire, pneumonie, tuberculose pulmonaire, bronchite, mal de gorge, amygdalite, sinusite paranasale, rhinite, bronchiolite constrictive et laryngite.

5. Composition pharmaceutique à utiliser selon la revendication 4, dans laquelle l'asthme est un asthme allergique causé par les acariens de la poussière, le pollen, les poils ou les squames d'animaux, les cafards, les aliments, les médicaments, la grippe, la fumée de cigarette, la contamination intérieure, la pollution de l'air, les additifs alimentaires, l'exercice, le changement climatique, la poussière jaune ou le stress.

6. Composition pharmaceutique à utiliser selon la revendication 5, dans laquelle l'asthme est un asthme allergique causé par les cafards.

7. Composition pharmaceutique selon la revendication 1 à utiliser selon la revendication 1, dans laquelle le sujet traité est un animal non humain.
